# EUROPEAN PATENT SPECIFICATION

(11) **EP 0 486 644 B1**
(45) Date of publication and mention of the grant of the patent: **19.07.1995**
(21) Application number: 91910729.2
(22) Date of filing: 12.06.1991
(51) Int. Cl.: A61F 2/38

(54) **PROSTHETIC KNEE JOINT DEVICES**
PROTHETISCHES KNIEGELENK
DISPOSITIFS PROSTHETIQUES POUR ARTICULATIONS DU GENOU

(30) Priority: 12.06.1990 GB 9013025
(43) Date of publication of application: 27.05.1992
(73) Proprietor: BRITISH TECHNOLOGY GROUP LIMITED, London SE1 6BU (GB)
(72) Inventor: GOODFELLOW, John, William, Oxford OX3 9TJ (GB); O'CONNOR, John, Joseph, Oxford OX3 8JN (GB)
(74) Representative: Parker, Geoffrey
(86) International application number: GB9100943
(87) International publication number: WO9119466

(56) References cited:
- EP-A- 0 183 670
- DE-A- 2 631 351
- GB-A- 1 534 263
- US-A- 4 216 549
- US-A- 4 257 129

## Description

This invention concerns prosthetic knee joint devices and more particularly such devices of the general kind described in Patent Specification GB-A-1534263.

Devices of this last kind involve a femoral component having a convexly curved articulation surface, a tibial component having a relatively flattened articulation surface, and a meniscal component having two articulation surfaces in back-to-back disposition and respectively substantially complementary to, and for mutual engagement with, the femoral and tibial component articulation surfaces. These devices can employ components of unicompartmental, bicompartmental or, in the case of the femoral component, tricompartmental form, or components in a combination of such forms. In all cases each component is normally of a one-piece construction to accord with the usual practice.

It is a feature of such a device that it can allow freedom of movement under the control of the related soft tissues in a natural manner and, at the same time, congruity between the engaged articulation surfaces. More specifically the freedom of movement should involve rotation at the femoral-meniscal interface and both rotation and translation at the meniscal-tibial interface. It is appropriate of course that movement should not lead to dislocation of the meniscal component but, given suitable design and application of the components, the component is normally retained in the desired manner between the associated components.

Even so, some forms of the device in current usage provide a mechanical coupling between the meniscal and tibial components with a view to ensuring the avoidance of dislocation while, at the same time, affording the desired mobility for the meniscal component. However, it is questionable whether this result can in fact be achieved fully satisfactorily by the couplings which have been proposed.

The couplings in question have commonly been of a tracked form involving a projection extending from one of the components into a recess in the other component. An immediate difficulty is that the freedom for movement is constrained and, in practice, congruity of articulation surface is degraded in compensation, but with consequent increase in contact stresses and component wear. There may also be a difficulty during surgery in locating the meniscal component between the femoral and tibial components and, at the same time, engaging the coupling without these last components being unduly spaced apart. This can lead to an incidence of undesirable laxity.

An object of the invention is to reduce the difficulties of this situation and, to this end, it is proposed that a device of the kind in question involving components of one-piece construction should include means separably connectable with the anterior peripheral part of the tibial component at least partially to pass over or into the associated meniscal component to retain the latter mutually engaged with said surface.

US-A-4216549 discloses a meniscal/tibial component assembly similar in some respects to that proposed here, but no translation can occur in the articulation movement. Also it involves a two-part tibial component/articulation surface structure.

As so far developed the invention is preferably applied to a device in which the meniscal and tibial components are each of one-piece bicompartmental construction having a pair of mutually spaced end portions respectively defining individual condylar articulation surfaces, such end portions being joined by way of a necked portion, and the separably connectable means including an elongate member at least partially to pass over the tibial component necked portion. The convenience of this arrangement is that the necked portions of the components are located in the intercondylar space and the elongate member can pass wholly over the meniscal component without impact on related articulation capabilities.

An alternative possibility is that the member can pass through, or into, the meniscal component. While this possibility is less convenient for biocompartmental components in that the meniscal component is a less simple structure, it offers the benefit of application to unicompartmental components.

In any event, the invention is clarified by way of example with reference to the accompanying drawings in which:-
Figures 1 and 2 respectively schematically illustrate in exploded and connected forms a presently preferred bicompartmental embodiment of an assembly according to the invention; and
Figure 3 similarly illustrates in connected form a unicompartmental embodiment.

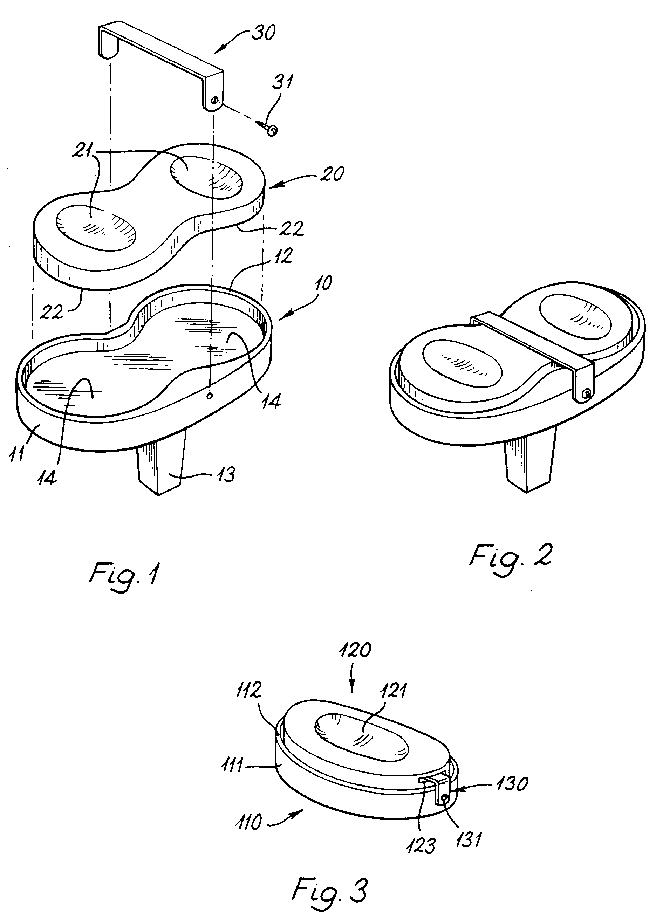

The assembly of Figures 1 and 2 comprises a bicompartmental tibial component 10 of one-piece construction including a main plate-like body 11 having a peripheral rim 12 upstanding from one major surface and at least one stem or other element 13 projecting from the other such surface. As seen from its major surfaces, the main body has two similar end portions joined by way of a necked portion. The one major surface is planar with the end portions thereof respectively defining individual condylar articulation surfaces 14. The other major surface and its projecting element can be formed in any manner appropriate for securement to bone.

The assembly of Figures 1 and 2 also comprises a bicompartmental meniscal component 20 of necked plate-like body shape similar to that of the tibial component. However, the body in this case is of smaller transverse dimensions over its major surfaces, and has one such surface 22 planar, whereby it can seat on the one surface of the tibial component and articulate, within the limits of the rim of the latter, by translation and rotation. Also, the meniscal component body thickness is greater than the height of the tibial component rim to extend above the latter when seated therein. The other major surface of the meniscal component has respective concave articulation surfaces 21 formed in its end portions for engagement with an associated femoral component (not shown).

Lastly in Figures 1 and 2, an elongate member 30 is provided. This member is of strip or other form having downwardly turned end portions and being dimensioned overall to bridge over the meniscal component, when this is seated in the tibial component, and engage its turned end portions with the tibial component. The member is secured in place by a screw 31 passed through one of the turned end portions into the tibial component.

It is to be noted that although the meniscal component will normally be located after the associated tibial and femoral components have been secured, and that the meniscal component must therefore pass over the tibial component rim, undue separation of the associated components is not necessary for this last purpose. The meniscal component can approach the site, with the leg fully flexed at the knee, from the front with its leading edge inclined downwardly within the upper tray-like formation of the tibial component, and then slid across this formation with a progressive downward rotation at its trailing edge to pass below and around the femoral component and fully seat in place. Thereafter the member 30 is conveniently located and secured from the front to act against meniscal component dislocation.

More particularly, it is to be noted that this securement against dislocation does not constrain the freedom for movement between the components in such a way as to require compensation by degraded congruity in articulatory engagement.

The assembly of Figure 3 is similar to one end portion of that of Figures 1 and 2, and employs the same reference numerals for corresponding parts and features, but with the addition of a one hundred digit for purposes of distinction. A clear difference in the unicompartmental form of Figure 3 is that the member 130 passes into a cavity 123 in, rather than over, the meniscal component 120.

While the invention has been described with more particular reference to the illustrated embodiments, it will be appreciated that other variations are possible. For example, it has been indicated that the separably connectable means need only partially bridge the meniscal component and this can entail variation in the extent to which the relevant member passes over or through the meniscal component. Also it will be evident that it is not necessary to have a rim wholly around the tibial component: the provision of short lengths of rim, or posts, at the front and rear of each condylar area can suffice to contain the meniscal movement relative to the tibial component within the appropriate controlled range.

## Claims

1. An assembly for a prosthetic knee joint device, said assembly comprising: a meniscal component (20) having two articulation surfaces (21,22) in back-to-back disposition, one (21) of such surfaces being of concavely curved form, and the other (22) of such surfaces being of relatively flattened form; and a tibial component (10) having an articulation surface (14) substantially complementary with said flattened surface; said concavely curved surface being engageable with a convexly shaped femoral articulation surface, said components each being of a one-piece construction, and said flattened and complementary surfaces being mutually engageable for articulation involving both rotation and translation; characterised by means (30) separably connectable with the anterior peripheral part of said tibial component (10), said means (30) at least partially passing over or into said meniscal component (20) to retain said mutual engagement.

2. An assembly according to Claim 1 wherein said means includes an elongate member (30) to extend over said meniscal component (20).

3. An assembly according to Claim 1 wherein said means includes an elongate member (130), and said meniscal component (120) is formed with a cavity (123) to receive said member.

4. An assembly according to Claim 1,2 or 3 wherein said components (20,10) are each of bicompartmental construction having a pair of mutually spaced end portions respectively defining individual condylar articulation surfaces (22,22; 14,14) for said engagement, such end portions being joined by way of a necked portion, and said means serving at least partially to pass over said meniscal component necked portion.

5. An assembly according to Claim 3 wherein said components (120,110) are each of unicompartmental construction defining a single condylar articulation surface for said engagement.

6. An assembly according to Claim 4 or 5 wherein said condylar articulation surfaces are substantially planar, and said tibial component (10, 110) has an upstanding peripheral rim formation (12, 112) bordering said surfaces to limit mutual articulation therebetween.

## Patentansprüche

1. Aufbau für ein prothetisches Kniegelenk, wobei dieser Aufbau umfaßt:
ein Meniskusteil (20), das zwei Rücken an Rücken angeordnete Gelenkflächen (21, 22) aufweist, wobei eine (21) dieser Flächen konkav gebogen und die andere (22) dieser Flächen relativ eben ausgebildet ist; und ein Tibia-Teil (10) mit einer Gelenkfläche (14), die im wesentlichen zu der ebenen Fläche komplementär ist; wobei die konkav gebogene Fläche mit einer konvex ausgebildeten Femur-Gelenkfläche verbindbar ist, wobei die genannten Teile jeweils einstückig sind und wobei die ebene und die komplementäre Fläche für eine Gelenkverbindung, die sowohl eine drehende als auch eine fortschreitende Bewegung einschließt, miteinander verbindbar sind;
gekennzeichnet durch eine Einrichtung (30), die abtrennbar mit dem vorderen Umfangsteil des Tibia-Teils (10) verbunden werden kann, wobei diese Einrichtung (30) zumindest teilweise über bzw. in das Meniskusteil (20) läuft, um die gegenseitige Verbindung zu sichern.

2. Aufbau nach Anspruch 1, wobei die Einrichtung ein langgestrecktes Element (30) aufweist, das sich über das Meniskusteil (20) erstreckt.

3. Aufbau nach Anspruch 1, wobei die Einrichtung ein langgestrecktes Element (130) aufweist und das Meniskusteil (120) mit einem Hohlraum (123) zur Aufnahme des Elements ausgebildet ist.

4. Aufbau nach Anspruch 1, 2 oder 3, wobei die Teile (20, 10) jeweils zwei Fächer aufweisen mit zwei zueinander beabstandeten Endbereichen, die jeweils einzelne Kondylus-Gelenkflächen (22,22; 14,14) für die Verbindung definieren, wobei diese Endbereiche durch eine Engstelle miteinander verbunden sind, und wobei die Einrichtung zumindest teilweise dazu dient, über diese Engstelle des Meniskusteils zu verlaufen.

5. Aufbau nach Anspruch 3, wobei die Teile (120, 110) jeweils ein Fach aufweisen, das eine einzelne Kondylus-Gelenkfläche für die Verbindung definiert.

6. Aufbau nach Anspruch 4 oder 5, wobei die Kondylus-Gelenkflächen im wesentlichen eben sind, und das Tibia-Teil (10, 110) einen aufrecht ausgebildeten umlaufenden Rand (12, 112) aufweist, der die Flächen eingrenzt, um eine gegenseitige Gelenkverbindung zwischen ihnen einzuschränken.

## Revendications

1. Ensemble pour un dispositif prothétique d'articulation de genou, ledit ensemble comportant : un composant méniscal (20) comportant deux surfaces d'articulation (21, 22) en disposition dos à dos, l'une (21) de ces surfaces ayant une forme incurvée de façon concave, et l'autre (22) de ces surfaces ayant une forme relativement aplatie ; et un composant tibial (10) comportant une surface d'articulation (14) sensiblement complémentaire de ladite surface aplatie; ladite surface incurvée de façon concave pouvant venir en contact avec une surface d'articulation fémorale de forme convexe, lesdits composants étant chacun d'une construction en une seule pièce, et lesdites surfaces aplatie et complémentaire pouvant venir en contact mutuel pour réaliser une articulation permettant aussi bien la rotation que la translation ; caractérisé par des moyens (30) pouvant être connectés séparément à la partie périphérique antérieure dudit composant tibial (10), lesdits moyens (30) passant au moins partiellement au-dessus ou à l'intérieur dudit composant méniscal (20) afin de maintenir ledit contact mutuel.

2. Ensemble selon la revendication 1, dans lequel lesdits moyens comprennent un élément allongé (30) destiné à s'étendre au-dessus dudit composant méniscal (20).

3. Ensemble selon la revendication 1, dans lequel lesdits moyens comprennent un élément allongé (130), et ledit composant méniscal (120) est constitué avec une cavité (123) destinée à recevoir ledit élément.

4. Ensemble selon la revendication 1, 2 ou 3, dans lequel lesdits composants (20, 10) ont chacun une construction bicompartimentale, et possèdent une paire de parties d'extrémité mutuellement espacées, définissant respectivement des surfaces d'articulation condylienne individuelles (22, 22 ; 14, 14) pour ladite venue en contact, ces parties d'extrémité étant réunies au moyen d'une partie de col, et lesdits moyens servant au moins partiellement à passer au-dessus de ladite partie de col de composant méniscal.

5. Ensemble selon la revendication 3, dans lequel lesdits composants (120, 110) ont chacun une construction unicompartimentale définissant une surface d'articulation condylienne unique pour ladite venue en contact.

6. Ensemble selon la revendication 4 ou 5, dans lequel lesdites surfaces d'articulation condylienne sont sensiblement planes, et ledit composant tibial (10, 110) comporte un rebord périphérique dirigé vers le haut (12, 112) bordant lesdites surfaces afin de imiter l'articulation mutuelle entre celles-ci.
